(19) Europäisches Patentamt
European Patent Office
Office européen des brevets

(11) **EP 4 379 665 A1**

(12) **EUROPEAN PATENT APPLICATION**

(43) Date of publication:
**05.06.2024 Bulletin 2024/23**

(21) Application number: **22210590.0**

(22) Date of filing: **30.11.2022**

(51) International Patent Classification (IPC):
**G06T 11/60** (2006.01)

(52) Cooperative Patent Classification (CPC):
**G06T 11/60**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventors:
• **BONDAR, Maria Luiza
Eindhoven (NL)**

• **NEUKIRCHEN, Christoph
Eindhoven (NL)**
• **HEESE, Harald Sepp
5656AG Eindhoven (NL)**
• **TORBJøRN, Vik
5656AG Eindhoven (NL)**
• **MOESSEL, Richard
Eindhoven (NL)**

(74) Representative: **Philips Intellectual Property &
Standards
High Tech Campus 52
5656 AG Eindhoven (NL)**

(54) **APPARATUS AND METHODS FOR VISUALISING IMAGING DATA**

(57) Aspects and embodiments relate to methods for mapping additional information relating to a 2D image of a region of interest to a 3D image volume of the region of interest and an apparatus and computer program product configured to perform such methods. A method according to an embodiment comprises: receiving a 2D composite image of the region of interest and a 3D image volume of the region of interest; decomposing the 2D composite image into at least: a base image; and an additional image comprising the additional information; identifying, based on an assessment of the base image and the 3D image volume, a representative 2D slice of the 3D image volume which corresponds to the base image; evaluating a transformation of the base image which maps the base image to the representative 2D slice; and combining the representative 2D slice with an image formed by applying the evaluated transformation to the additional image to produce a new composite 2D image of the region of interest.

Aspects and embodiments recognize that it is possible to utilize information provided in a 2D image and map that information onto new imaging information forming part of a 3D image volume. Providing a visualization of such a mapping can assist an imaging professional to more efficiently evaluate captured image data representative of a region of interest. In particular, the mapping of additional information to a portion of a 3D image volume of a region of interest may assist an imaging professional to perform the technical task of evaluating the clinical relevance of the captured 3D image data. That evaluation may be performed for the purpose of determining an appropriate course of treatment, or for evaluating or diagnosing a disease or chronic condition on the basis of captured image data.

FIG. 2

EP 4 379 665 A1

**Description**

TECHNOLOGICAL FIELD

Various example embodiments relate to imaging data. In particular, aspects and embodiments relate to an apparatus and method for mapping or visualizing additional information relating to a 2D image of a region of interest to a 3D image volume of the region of interest.

BACKGROUND

Patients experiencing a chronic condition or disease may be subject to one or more treatment and associated follow-on treatment or aftercare. Initial investigation, treatment and consequent follow up processes may be understood to represent a series of substantially independent interactions with a subject and each interaction can be associated with capture or creation of one or more image, or set of imaging data, associated with the subject and the condition or disease of interest.

By way of example, cancer patients may experience initial investigations, and may also typically undergo follow-up imaging studies at a radiology department after receiving cancer therapy. Such studies can be performed for the purpose of tumor response assessment and/or assessment of tissue changes after receipt of cancer therapy.

When imaging a patient, often imaging methods support capture of 3D imaging data.

In the case of cancer treatment, for example, imaging data relating to, for example, previous tumor assessment or examination and imaging data which captures details of previous investigation or treatment (for example, radiotherapy dose) can be useful to various healthcare professionals interacting with a patient. For example, correlation of equivalent 3D imaging data from previous assessment(s) or treatment(s) with imaging data being acquired can support, for example, measurement or assessment of tumor change. Such a direct correlation between 3D imaging data sets may also, for example, allow a radiologist to more easily differentiate between tumor progression and radiation therapy side effects.

Technically, the task of "correlation" or image labelling by dose level can be solved by performing a non-rigid registration between a follow up 3D image (CT, MRI, etc) and a previously acquired 3D planning image, and by warping known delivered dose information (or dose levels) which are known in relation to the 3D planning image to the geometry of the follow-up 3D image. Similarly, 3D image registration can be used to correlate findings in a current 3D medical image to findings in a previously acquired 3D medical image.

Although technically feasible, such registration between a contemporaneous follow-up 3D imaging data set acquired at a radiology practice to previously acquired planning 3D imaging data at a radiotherapy department can be rendered difficult by various factors, including, for example, data access limitations between departments, and/or between different institutes that may not share the same image data archive. Furthermore, even within the same hospital, search and retrieval of previous 3D CT and radiotherapy dose distribution data from a PACS archive can be slow.

Savjani et al "A Framework for Sharing Radiation Dose Distribution Maps in the Electronic Medical Record for Improving Multidisciplinary Patient Management" [Radiol Imaging Cancer 2021 Mar 12;3(2):e200075. doi: 10.1148/rycan.2021200075. eCollection 2021 Mar] recognizes that the dedicated software and hardware of a radiation oncology practice may result in radiation treatment history being inaccessible to other medical subspecialties. The reference notes difficulties which occur if 3D radiotherapy data specific data from the radiotherapy department is pushed to a picture archiving and communicating system (PACS) of a hospital and notes that raw radiotherapy data is not readable by a PACS provided at most hospitals.

Some adaptations to processes and methodologies relating to sharing of imaging data may support creation of information of ongoing use to medical professionals by offering ways to combine previously acquired information with a new imaging data set.

SUMMARY

The scope of protection sought for various example embodiments of the invention is set out by the independent claims. The example embodiments and features, if any, described in this specification that do not fall under the scope of the independent claims are to be interpreted as examples useful for understanding various embodiments of the invention.

According to various, but not necessarily all, example embodiments there is provided an apparatus configured to map additional information relating to a 2D image of a region of interest to a 3D image volume of the region of interest, the apparatus comprising: at least one processor; and at least one memory storing instructions that, when executed by the at least one processor, cause the apparatus at least to: receive a 2D composite image of the region of interest; receive a 3D image volume of the region of interest; decompose the 2D composite image into at least: a base image; and an additional image comprising the additional information; identify, based on an assessment of the base image and the 3D image volume, a representative 2D slice of the 3D image volume which corresponds to the base image; evaluate a transformation of the base image which maps the base image to the representative 2D slice; and combine the representative 2D slice with an image formed by applying the evaluated transformation to the additional image to produce a new composite 2D image of the region of interest.

In one embodiment, the 2D image of a region of interest comprises: a color image.

In one embodiment, the composite 2D image comprises: a 2D summary image of a 3D image volume representative of the region of interest.

In one embodiment, the composite 2D image comprises: additional information relating to a treatment or procedure performed upon the region of interest.

In one embodiment, wherein the additional information comprises: a geometrical distribution of a therapy dosage applied to the region of interest.

In one embodiment, the therapy comprises radiotherapy.

In one embodiment, the geometrical distribution comprises: isodose lines or color mapped dosage information.

In one embodiment, the additional information comprises: one or more annotation comprising: an outline of a feature of interest; and/or a marker of a feature of interest; and/or a measurement of a feature of interest.

In one embodiment, decomposing the 2D composite image comprises: decomposing the 2D composite image into at least: a greyscale base image; and an additional image comprising the additional information.

In one embodiment, decomposing the 2D composite image comprises: analysis of pixels of the 2D composite image to determine the greyscale base image and an additional image comprising the additional information

In one embodiment, identifying a representative 2D slice of the 3D image volume which corresponds to the base image comprises: assessing one or more plane image forming the 3D image volume against the base image and selecting a plane image of the assessed one or more plane image that best matches the base image.

In one embodiment, identifying the representative 2D slice of the 3D image volume and evaluating a transformation of the base image which maps the base image to the representative 2D slice is determined as a combined calculation.

In one embodiment, identifying the representative 2D slice of the 3D image volume and evaluating a transformation of the base image which maps the base image to the representative 2D slice comprises: using a slice to volume or 2D to 2D registration image registration technique.

In one embodiment, evaluating a transformation of the base image which maps the base image to the representative 2D slice comprises evaluating one or more difference between those images resulting from one or more of: rotation, translation, deformation, or scaling.

According to various, but not necessarily all, example embodiments there is provided a computer implemented method, for mapping additional information relating to a 2D image of a region of interest to a 3D image volume of the region of interest, the method comprising: receiving a 2D composite image of the region of interest and a 3D image volume of the region of interest; decomposing the 2D composite image into at least: a base image; and an additional image comprising the additional information; identifying, based on an assessment of the base image and the 3D image volume, a representative 2D slice of the 3D image volume which corresponds to the base image; evaluating a transformation of the base image which maps the base image to the representative 2D slice; and combining the representative 2D slice with an image formed by applying the evaluated transformation to the additional image to produce a new composite 2D image of the region of interest.

In one embodiment, the 2D image of a region of interest comprises: a color image.

In one embodiment, the composite 2D image comprises: a 2D summary image of a 3D image volume representative of the region of interest.

In one embodiment, the composite 2D image comprises: additional information relating to a treatment or procedure performed upon the region of interest.

In one embodiment, wherein the additional information comprises: a geometrical distribution of a therapy dosage applied to the region of interest.

In one embodiment, the therapy comprises radiotherapy.

In one embodiment, the geometrical distribution comprises: isodose lines or color mapped dosage information.

In one embodiment, the additional information comprises: one or more annotation comprising: an outline of a feature of interest; and/or a marker of a feature of interest; and/or a measurement of a feature of interest.

In one embodiment, decomposing the 2D composite image comprises: decomposing the 2D composite image into at least: a greyscale base image; and an additional image comprising the additional information.

In one embodiment, decomposing the 2D composite image comprises: analysis of pixels of the 2D composite image to determine the greyscale base image and an additional image comprising the additional information

In one embodiment, identifying a representative 2D slice of the 3D image volume which corresponds to the base image comprises: assessing one or more plane image forming the 3D image volume against the base image and selecting a plane image of the assessed one or more plane image that best matches the base image.

In one embodiment, identifying the representative 2D slice of the 3D image volume and evaluating a transformation of the base image which maps the base image to the representative 2D slice is determined as a combined calculation.

In one embodiment, identifying the representative 2D slice of the 3D image volume and evaluating a transformation of the base image which maps the base image to the representative 2D slice comprises: using a slice to volume or 2D to 2D registration image registration technique.

In one embodiment, evaluating a transformation of the base image which maps the base image to the representative 2D slice comprises evaluating one or more difference between those images resulting from one or more of: rotation, translation, deformation, or scaling.

According to various, but not necessarily all, example embodiments there is provided a computer program product operable, when executed on a computer, to perform a method for mapping additional information relating to a 2D image of a region of interest to a 3D image volume of the region of interest, the method comprising: receiving a 2D composite image of the region of interest and a 3D image volume of the region of interest; decomposing the 2D composite image into at least: a base image; and an additional image comprising the additional information; identifying, based on an assessment of the base image and the 3D image volume, a representative 2D slice of the 3D image volume which corresponds to the base image; evaluating a transformation of the base image which maps the base image to the representative 2D slice; and combining the representative 2D slice with an image formed by applying the evaluated transformation to the additional image to produce a new composite 2D image of the region of interest.

According to various, but not necessarily all, example embodiments there is provided a non-transitory computer-readable medium storing computer program code including instructions that, when executed by a processor, cause a computer to perform a method for mapping additional information relating to a 2D image of a region of interest to a 3D image volume of the region of interest, the method comprising: receiving a 2D composite image of the region of interest and a 3D image volume of the region of interest; decomposing the 2D composite image into at least: a base image; and an additional image comprising the additional information; identifying, based on an assessment of the base image and the 3D image volume, a representative 2D slice of the 3D image volume which corresponds to the base image; evaluating a transformation of the base image which maps the base image to the representative 2D slice; and combining the representative 2D slice with an image formed by applying the evaluated transformation to the additional image to produce a new composite 2D image of the region of interest.

Some embodiments recognize that it is possible to utilize information provided in a 2D image and map that information onto new imaging information forming part of a 3D image volume. Providing a visualization of such a mapping, in the form of a new composite 2D image, can assist an imaging professional to more efficiently evaluate captured image data representative of a region of interest.

In particular, embodiments recognize that the mapping of additional information to a portion of a 3D image volume of a region of interest may assist an imaging professional to perform the technical task of evaluating the clinical relevance of the captured 3D image data. That evaluation may be performed for the purpose of determining an appropriate course of treatment, or for evaluating or diagnosing a disease or chronic condition on the basis of captured image data.

These and other aspects of the invention will be apparent from and elucidated with reference to the embodiment(s) and arrangement(s) described hereinafter.

Further particular and preferred aspects are set out in the accompanying independent and dependent claims. Features of the dependent claims may be combined with features of the independent claims as appropriate, and in combinations other than those explicitly set out in the claims.

Where an apparatus feature is described as being operable to provide a function, it will be appreciated that this includes an apparatus feature which provides that function or which is adapted or configured to provide that function.

BRIEF DESCRIPTION OF THE DRAWINGS

Some example embodiments will now be described with reference to the accompanying drawings in which:

FIG. 1A and FIG. 1B show two example 2D images of a region of interest of a subject;
FIG. 2 illustrates schematically input and output of a methodology according to an arrangement;
FIG. 3 illustrates schematically input and output of a methodology according to a further arrangement;
FIG. 4 illustrates schematically a system for use in accordance with one arrangement; and
FIG. 5 illustrates schematically main steps of a method according to an arrangement.

DETAILED DESCRIPTION

Before discussing the example embodiments in any more detail, first the context within which aspects and embodiments are to be understood is provided.

Patients experiencing a chronic condition or disease may be subject to one or more interaction with various healthcare professionals. Initial investigation, treatment and consequent follow-up processes may be understood to represent a series of substantially independent interactions with a patient and each interaction can be associated with capture or creation of one or more image, or set of imaging data, associated with the patient and the condition or disease of interest.

By way of example, cancer patients may experience initial investigations including imaging of a region of interest, treatment of the region of interest, which may be planned, or occur using imaging processes, and may also typically undergo follow-up imaging studies at, for example, a radiology department after receiving cancer therapy.

Ongoing imaging study of a patient can be performed for various purposes, including: monitoring of progression of a disease, tumor response assessment and/or assessment of tissue changes after receipt of cancer therapy.

When imaging a patient, often imaging methods used support capture of 3D imaging data, in the form of a 3D image volume. Examples of 3D imaging methods include computed tomography scanning (CT scans) and magnetic resonance imaging scanning (MRI scans). Such imaging techniques allow capture of a 3D dataset or image volume relating to a region of a body.

In the case of cancer treatment, for example, CT and/or MRI scans may be performed to capture information relating to tumor assessment or examination. That CT or MRI imaging data may include detail relating to previous investigation or treatment (for example, radiotherapy dose) which is useful to various healthcare professionals who continue to interact with a patient.

By way of example, correlation of equivalent 3D imaging data from previous assessment(s) or treatment(s) with new 3D imaging data being acquired can support, for example, measurement or assessment of tumor change. Such a direct correlation between 3D imaging data sets may, for example, allow a radiologist to more easily differentiate between tumor progression and radiation therapy side effects.

Technically, the task of "correlation" or image labelling by dose level can be solved by performing a non-rigid registration between a newly acquired 3D image (CT, MRI, etc) and a previously acquired 3D planning image, and by warping known delivered dose information (or dose levels) which are known in relation to the 3D planning image to the geometry of the follow-up 3D image. Similarly, 3D image registration can be used to correlate findings in a current 3D medical image to findings in a previously acquired 3D medical image.

Although technically feasible, such registration between new 3D imaging data set and previously acquired 3D imaging data (for example, registration of a new imaging data set a dataset used to plan radiation therapy dose) at a radiotherapy department can be rendered difficult by various factors, including, for example, data access limitations between departments, and/or between different institutes that may not share the same image data archive. Furthermore, even within the same hospital, search and retrieval of previous 3D CT and radiotherapy dose distribution data from a PACS archive can be slow.

Complete 3D data collected previously may be unavailable, or subject to practical constraints. However, some simpler information summarizing detail of previously acquired imaging data, together with detail of applied treatment, for example, may be available when performing a subsequent study. By way of example, some 2D information, for example, an image or similar, summarizing detail of previously acquired imaging data together with a delivered radiotherapy plan is typically made available and associated with records held in relation to a patient.

Information relating to a patient can be made available to various parties or healthcare professionals as a patient progresses and is treated or monitored. Information summarizing detail of, for example, a treatment performed on a patient, may comprise a document, in soft or hard copy.

The information summary held in relation to a patient may comprise one or more summary 2D image of a region of interest. The summary image(s) may comprise RGB images of representative axial, coronal, and sagittal slices of, for example, 3D planning imaging data. Such imaging data, for example, may comprise 3D computerized tomography (CT) data acquired in relation to a patient. The summary 2D image(s) of a region of interest provided in an information summary may be fused with additional information relating to a treatment or procedure performed in relation to a patient. One example of such treatment-related information comprises: a geometrical distribution of planned radiotherapy dose. Such dosage information may, for example, comprise color coded isodose lines, or a color mapping representative of radiation dosage. The color mapping allows a user to visualize a dose on the basis of a color coded map. The color coded map assigns color(s) to different dosage ranges. The color mapping may comprise a color gradient which correlates with dosage. The summary documents may be prepared by a radiation oncologist at the end of a treatment. The summary documents are prepared for the purpose of communicating treatment or procedure details to other disciplines and departments.

Similarly, representative 2D images of previous 3D imaging events, for example, tumor assessment exams, can be summarized in one or more document, screen shot, or stored in an Electronic Medical Record (EMR) as one or more 2D image in a standard format (for example, jpeg). The representative 2D images of previous imaging occasions may include various additional information, for example, they may be annotated with tumor region of interest (ROI) contours; tumor size measurements (for example, lines marking tumor diameter, arrows, text, or similar). The 3D medical images in a Picture Archiving and Communication System (PACS) archive typically do not contain such annotations. For a medical professional, for example, a radiologist, performing a subsequent study to acquiring further imaging data, it can be time consuming to load any 3D images of previous examinations or studies from the PACS archive, reproduce previous measurements which may have been provided in a 2D format, and correlate any current findings with such previous measurements.

Whilst in principle it may seem possible for an imaging, or other healthcare professional to combine information from two sources (the newly acquired 3D dataset and a 2D summary of a previous investigation, treatment or intervention) just by looking at those sources of information, it will be appreciated that the complexity of the information and imaging data being acquired, together with changes in anatomy, for example, rotation, translation, and/or deformation, different scaling, and so on, means it can be difficult for a user to directly correlate the treatment dose or other information from the summary representative 2D images, or information provided in relation to previously acquired imaging investigations, with 3D patient anatomy in a follow up CT or MRI scan.

Having now described the context within which aspects and embodiments are to be understood, an overview of methodologies in accordance with some possible implementations is provided:

Arrangements may provide a method for mapping additional information relating to a 2D image of a region of interest to a 3D image volume of the region of interest. Arrangements may provide an apparatus configured to perform such a method. The apparatus may comprise a computer, for example, in the form of at least one processor; and at least one memory storing instructions that, when executed by the at least one processor, cause the computer to perform the method.

One method according to an arrangement comprises the steps of: receiving a 2D composite image of a region of interest and a 3D image volume of the region of interest. The method may comprise the step of decomposing the 2D composite image into at least: a base image; and an additional image comprising the additional information. The method may comprise the step of identifying, based on an assessment of the base image and the 3D image volume, a representative 2D slice of the 3D image volume which corresponds to the base image. The method may comprise the step of evaluating a transformation of the base image which maps the base image to the representative 2D slice. The method may comprise the step of combining the representative 2D slice with an image formed by applying the evaluated transformation to the additional image to produce a new composite 2D image of the region of interest.

In general, implementations recognize that it is possible to utilize information provided in a 2D image and map that information onto new imaging information forming part of a 3D image volume. Providing a visualization of such a mapping, in the form of a new composite 2D image, can assist an imaging professional to more efficiently evaluate captured image data representative of a region of interest. In particular, the mapping of additional information to a portion of a 3D image volume of a region of interest may assist an imaging professional to perform the technical task of evaluating the clinical relevance of the captured 3D image data. That evaluation may be performed for the purpose of determining an appropriate course of treatment, or for evaluating or diagnosing a disease or chronic condition on the basis of captured image data.

As described previously, aspects and embodiments recognize that an information summary held in relation to a patient may comprise one or more summary 2D image of a region of interest. The summary image(s) may comprise RGB images of representative axial, coronal, and sagittal slices of, for example, 3D planning imaging data. Such imaging data, for example, may comprise 3D computerized tomography (CT) data acquired in relation to a patient. The summary 2D image(s) of a region of interest provided in an information summary may be fused with additional information relating to image assessment, and/or a treatment or procedure performed in relation to a patient. One example of such treatment-related information comprises: a geometrical distribution of planned radiotherapy dose. Such dosage information may, for example, comprise color coded isodose lines, or a color gradient radiation dosage. The summary documents may be prepared by a radiation oncologist at the end of a treatment. The summary documents are prepared for the purpose of communicating treatment or procedure details to other disciplines and departments.

Aspects and embodiments recognize that it is possible to utilize such summary information, comprising 2D information, in conjunction with, for example, a newly acquired 3D imaging data set to provide a user, for example, a healthcare professional, with useful information to augment and allow evaluation of the newly acquired 3D imaging dataset. The useful information may comprise augmented imaging data offering a user assistance to interpret the newly acquired 3D imaging data. For example, the augmented imaging data may assist a user in relation to identification of and differentiation of tissue change(s) within a radio therapy treated area compared to tissue change(s) outside the radio therapy treated area.

Similarly, aspects and embodiments recognize that representative 2D images of previous 3D imaging events, for example, tumor assessment exams, can be summarized in one or more document, screen shot, or stored in an Electronic Medical Record (EMR) as one or more 2D image in a standard format (for example, jpeg). The representative 2D images of previous imaging occasions may include various forms of additional information which is not part of raw obtained image data. For example, additional information included in a 2D image of a region of interest may comprise one or more annotations indicative of a tumor region of interest (ROI) contour or outline; tumor size measurements (for example, lines marking tumor diameter, arrows, text, or similar).

Aspects and embodiments recognize that it is possible to utilize summary information relating to previous imaging events, comprising 2D information, in conjunction with a newly acquired 3D imaging data set to provide a user with useful information. The useful information may comprise augmented imaging data offering a user assistance to interpret the newly acquired 3D imaging data. For example, the augmented imaging data may assist a user in relation to tumor progression or similar, even when a treatment has not occurred.

Aspects and embodiments provide an apparatus and methodologies which support visualization, on a portion of a subsequently acquired 3D imaging dataset, of information derived from 2D images summarizing previous findings and/or treatment. Such information may, for example, comprise one or more geometrical feature relating to a previous finding and/or treatment. Representative 2D image(s) including additional information may comprise part of a treatment summary report or be summary information relating to previous imaging occasions. The representative 2D images may comprise one or more image provided in a record associated with a patient or imaging subject. The representative image may comprise an image stored in a standard image format in an Electronic Medical Record (EMR) system.

Thus, according to some arrangements, 2D imaging information, a 2D imaging dataset and/or information which maps to a 2D image can be received or otherwise obtained so that information relating to such a 2D image can be used in relation to a 3D imaging volume captured in relation to a similar region of interest.

In order to utilize additional information provided in, or relating to, a 2D image, it may be necessary to decompose the 2D image into at least two parts. Those parts may comprise: base image data and additional information which does not form part of the base image data.

According to one example arrangement, a representative 2D RGB summary image comprising, an image of a region of interest including additional information and/or annotations color dose distribution can be decomposed into:

> a grey scale slice (S) of a planning image and
> additional features, for example, color geometric dose features (GDF)

According to one arrangement, color (for example, isodose, or tumor extent) lines present in a representative 2D summary image can be extracted, for example, using a simple RGB decomposition of the picture and detecting pixels with non-equal R, G, and B values. In some examples, transparent color patches of alpha blended images can be extracted using mathematical optimization techniques. Accordingly, the summary image may be appropriately split into at least two images, with one image relating to a base image of a region of interest and a further image which comprises additional information relating to that base image.

Having decomposed the summary image into at least two images, according to some arrangements, a transformation (T) is calculated which maps the base image, for example, a grey scale slice of a planning image (S), to a substantially equivalent plane or slice of a 3D dataset acquired in the form of, for example, a 3D volume or follow up image. The 3D imaging dataset V may, for example, comprise new CT or MRI information obtained in relation to a region of interest.

According to some arrangements, the transformation is selected or evaluated such that a plane P or slice of the 3D dataset V is found which is found to substantially correspond to the 2D base image data. That evaluation, for example, may comprise evaluation of each plane P in volume V which could correspond to representative slice S. The evaluation may be such that it aims to determine an index of a slice (or plane) in V that best matches the base image.

The transformation T can be determined using, for example, slice to volume registration techniques, or simple 2D to 2D image registration techniques. Such image registration techniques may accommodate transformation of the base image to align with the plane of the image volume which best matches the base image to take into account: one or more difference between those images resulting from one or more of: rotation, translation, deformation, or scaling.

According to some arrangements, having determined a mapping or transformation which correlates the base image of the 2D image to a plane or slice of the 3D image dataset, that mapping can be applied to the additional information image. The additional information image may, for example, comprise an image including representative 2D geometric dosage features (GDF), or other additional information, for example, one or more indication of size, location, measurement or similar relating to one or more feature included in the base image. In other words, according to some arrangements, a mapping of the additional information available in relation to the summary 2D image can be applied such that the additional information can be equivalently related to an appropriate plane or section of a 3D imaging data set. Accordingly, in some arrangements, T(GDF) is evaluated.

According to some arrangements, a transformation of the additional information image can be combined with the plane or slice of the 3D dataset which has been determined to best correspond to the base image data. In one example, an appropriately transformed or mapped additional information image may be overlaid or combined with the plane or slice of the 3D dataset which has been determined to be substantially equivalent to the base image of the 2D summary image. In some arrangements, for example, T(GDF) is overlaid onto P, thereby allowing direct visualization of, for example, dose features on P. Similarly, by overlaying T(GDF) onto P, the P image can be viewed by a medical professional and include planned dose level information.

Decomposition of 2D Image

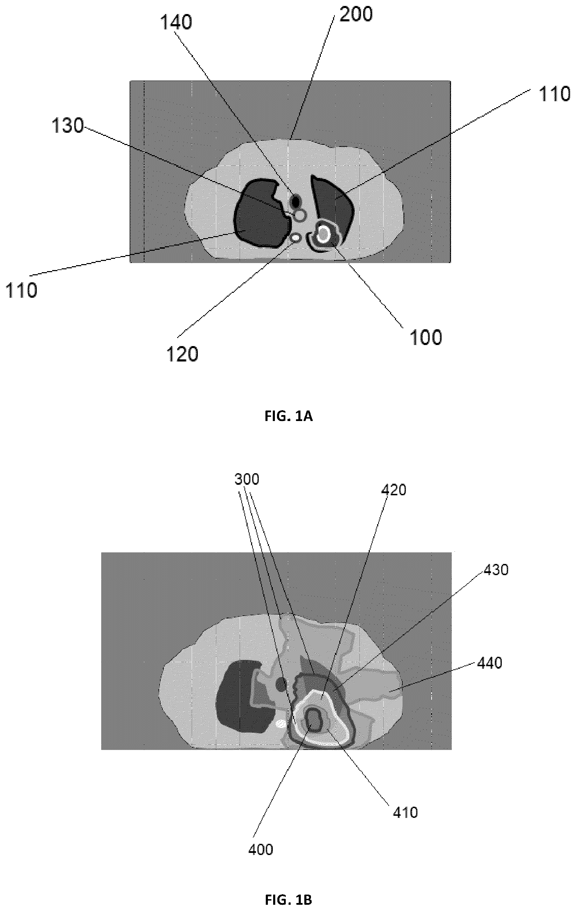

FIG. 1A and FIG. 1B show two example 2D images of a region of interest of a subject.

FIG. 1A shows a schematic representation of an image of a slice of a subject torso. The 2D image of FIG. 1A shows schematically an image which may result from a subject with histologically proven non-small cell lung carcinoma. The carcinoma is marked on the image as a contour 100. The general region of interest is indicated by outline 200 (the entire torso). Also marked on the image of FIG. 1A are various other features of interest, including lungs 110, spinal cord 120, oesophagus 130 and trachea 140.

FIG. 1B shows the slice of a subject torso of FIG. 1A, on which the dose distribution of a radio therapy treatment plan is shown. The treatment plan may, for example, comprise a Stereotactic Body Radiation Therapy plan.. The treatment plan relates to VMAT technology, 5 Gy in 10 fractions, giving a total dose of 50Gy. Isodose lines (% of total dose) 300 are shown and the color overlay shows the dose distribution: regions 400, 410 comprising a high dose regions surrounding the tumor, step down dosage regions 420, 430 and large low-dose region 440.

It will be understood that either of FIG. 1A or FIG. 1B can be used as a 2D image which includes both base image data (the grey scale image data set) and additional information. FIG. 1A includes additional information in relation to outlines of features of interest. FIG. 1B includes additional information in relation to outlines of features of interest and additional information relating to a treatment plan, namely in this instance: isodose lines and color scale dosage information overlaid onto the base image data.

According to arrangements, composite images such as those shown in FIG. 1A and 1B are "unblended" or "decomposed" to create at least two separate images.

It can be understood that the images shown in FIG. 1A and 1B include base image data in the form of a slice image of interest which was produced from a 3D CT image dataset. In the case of FIG. 1B, that base image data has been overlaid at some given transparency (i.e. alpha blended) with at least a second image. The second image represents, in this instance, a planned or intended radiation therapy treatment dose. Typically, the base image (in this case a CT image) comprises a grey scale image; the treatment dose image, or additional information, included in the composite image may typically comprise one or more color features or a color image.

In order to address image decomposition (i.e. unblending) if presented with a composite image such as those shown in FIG. 1A or 1B, a goal is set to obtain a version of the two original images from the single overlaid composite image.

In general, such decomposition may only be possible if certain assumptions regarding properties of the two original images are made. In a generic configuration, the color values of the pixels in each of the images involved are represented as an RGB-triple. The mapping of the CT intensity to the corresponding grey in each pixel of the grey scale image is realized by a grey scale color map that is assumed known. The mapping of radiation therapy dose levels to image color pixels is realized via an unknown RGB color mapping.

Accordingly, one possible approach to implement decomposition of an overlaid RGB image on the greyscale base image, can based on estimation of the unknown color mapping (as used for generating the original dose planning image) from the overlay image pixel data. Such color map estimation may be straightforward for color maps implemented in a subspace which is orthogonal to the grey scale of the CT image. For other color map subspaces such estimation is more complex and cannot generally be uniquely solved.

In practice, most commonly used RGB color maps represent a connected curve (e.g. a polygonal line) in RGB space. By assuming a general linear colormap (or a set of multiple linear maps) some proper statistical methods for estimation of vector space basis sets (for example, based on independent component analysis (ICA)) might be applied to estimate color maps as basis vectors from the image pixels' RGB values. If the color map must be assumed non-linear, some more sophisticated non-linear vector space estimation methods are required.

In the general case, when no assumptions on the underlying color maps (or other image properties) can be made, solving the image unblending problem may be impractical. However, the more information is given (for example, one or both colormaps might be known, or a colormap's subspace may be provided tightly) the more tractable the image unblending becomes.

Evaluation of Transformation

The problem of finding a corresponding cross section slice for a 2D slice image in a 3D volume can be formulated as a slice-to-volume registration problem. Given a 2D slice image S and a 3D volume V, slice-to-volume registration methods operate to identify a 2D-to-2D transformation function $\hat{T}$, and a plane $\hat{P}$ [V] (i.e., a 2D slice from the volume V).

In the most general case, $\hat{T}$ and $\hat{P}$ minimize the following objective function:

$$\widehat{T}, \widehat{P} = \underset{T,P}{\operatorname{argmin}}\, C(S \circ T(x), P[V](x)) + R(T, P)$$

Where:

$\widehat{T}$ is an "optimal" transformation, and
P is an "optimal" plane;
C is an image similarity function, and
R is a regularization term.

The mapping T may be rigid, affine, or non-rigid. The image similarity function C is a quantitative evaluation of similarity between the transformed 2D image and the corresponding slice in the 3D volume V.

Various types of image similarity functions C can be used, for example, similarity functions defined using only the intensity values in S and V (e.g., normalized cross correlation, mutual information), or defined using only geometric landmarks, or a combination of both image intensity values and geometric landmarks in S and V.

The regularization term R can be used to render the problem well posed. The regularizer R may impose constraints to the solution (e.g., to minimize out-of-plane deformations, minimize deformation magnitude, impose elasticity constraints, etc.).

Alternatively, the problem of finding a corresponding cross section slice for a 2D image in a 3D volume can be formulated as an iterative 2D registration problem, where the 2D image is iteratively registered with slices of the 3D volume. The best or optimal corresponding slice can be identified by the index of the slice found to have the lowest value of similarity function.

It will therefore be appreciated that the steps of identification of a representative slice and evaluation of a transformation may be combined or may be performed separately.

By implementing methodologies in line with those described above, arrangements facilitate continued interaction of a user with new imaging data into which information from a summary has been combined. A user may, for example, interact with the output of some arrangements via a graphical user interface (GUI). Arrangements allow direct user interaction with a least one of: a 2D image or images provided as a summary of previous or planned treatment or investigation; or a transformed image according to which an equivalent plane or slice of a 3D imaging dataset is identified as equivalent to the 2D image or images; or a transformed additional information image comprising information relating to the 2D image or images, including, for example, geometrical features of a delivered dose, and/or transformed measurements (lines, regions of interest). A user may be able to interact with, and activate or deactivate overlaying of various available 2D images including:

the original 2D image(s);
an equivalent transformed image in the form of a plane or slice of a 3D imaging dataset which has been evaluated to be equivalent to the 2D image or images;
transformed additional information image features which were included in the original 2D image(s).

Accordingly, arrangements and implementations may provide a system and methodology to support tools to visualize additional information, for example, one or more geometrical feature, measurement, annotation or similar relating to previous findings and/or treatment(s), in relation to a newly acquired imaging dataset of the same region of interest. The additional information may form part of a representative 2D image, or set of images, forming part of a treatment or findings summary report in an electronic medical record system. The 2D image or set of images may comprise one or more file stored in a standard image format, for example .jpeg or similar.

Arrangements may allow a user, for example, a medical imaging professional, to make a faster assessment of newly acquired imaging information based on a combination of the newly acquired imaging information with a summary report of previous findings or treatment provided in relation to one or more representative slice or plane of a region of interest.

Example One: Treatment Summary

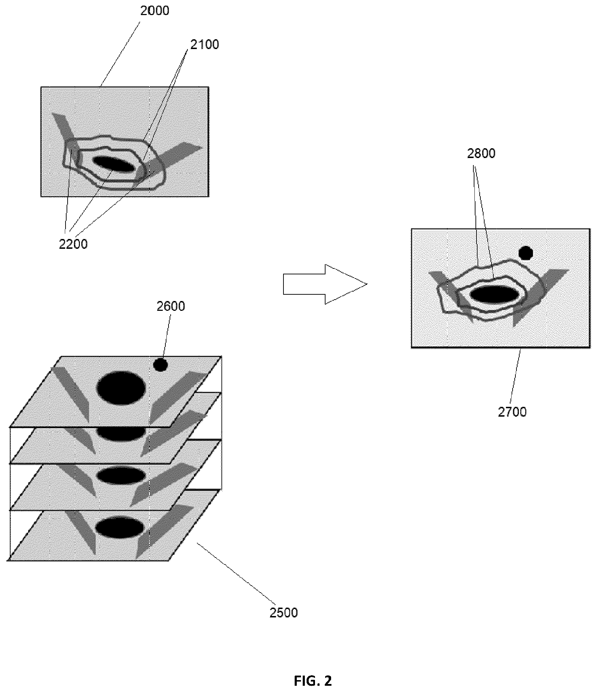

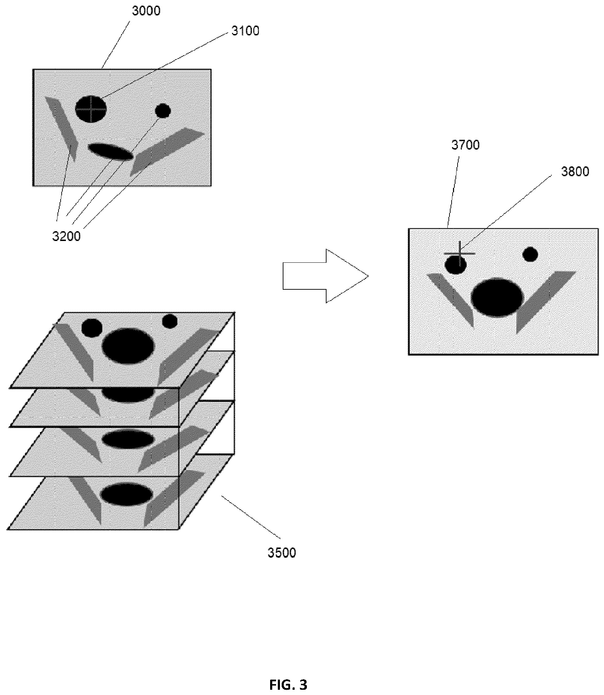

FIG. 2 illustrates schematically input and output of a methodology according to an arrangement. Having described methodologies in general, a specific implementation in relation to application of such methodologies for the case of representative 2D images from radiotherapy treatment or 2D images with annotations from previous tumor assessment examination (as described in relation to FIG. 3) is now described in more detail:

FIG. 2 shows a representative 2D image 2000 which can be extracted from storage medium. The 2D image may comprise a part of a patient report, a screen grab, part of an electronic medical record EMR or similar. In this instance, the 2D image may comprise a treatment plan summary. The image 2000 comprises a composite image formed from base image data and a treatment plan, relating to radiotherapy. The treatment isodose lines 2100 cane be seen to form part of image 2000. The base image includes one or more features 2200 of the region under study

According to a methodology in accordance with an arrangement, the representative 2D RGB image 2000 (of, for example, a previously acquired CT including color dose distribution) is decomposed into (i) a grey scale slice (S) representing the planning image and (ii) color geometric dose features (GDF). For example, colored isodose lines can be extracted from a representative 2D RGB image using a simple RGB decomposition of the 2D RGB image and detecting pixels with non-equal R, G, and B values. Transparent color patches of alpha blended images can be extracted using appropriate mathematical optimization techniques.

FIG. 2 illustrates schematically a volume 2500 of 3D imaging data which is available in relation to a region under study. The volume of image data 2500 may comprise, for example a series of image "slices" obtained via appropriate CT or MRI techniques.

According to methods in accordance with arrangements, base image data from the 2D image 2000 and the 3D volume of the follow up image are processed so that a transformation T can be calculated. T maps S, the base image data in relation to 2D image 2000, into a 3D volume 2500 of a follow up image (V). T is calculated based upon a determination of a plane P in V which may be considered to correspond to representative slice S. The transformation T can be determined using slice to volume registration, or simple 2D to 2D registration, each technique operating with an aim of determining an index of a slice in V that best matches S. It will be appreciated that it may be impossible to find a slice of V 2500 which completely matches the features 2200 of the base image data of 2D image 2000. That may be because capture of the 2D image and 3D volume of image data is spaced apart in time and changes in features 2200 have occurred. In the example shown in FIG. 2, for example, an additional feature 2600 is present. Nonetheless, in accordance with arrangements, a best fit slice or plane of the image data 2500 is found.

Once calculated, T can then be applied to the representative 2D color geometric dosage features 2100 of 2D image 2000 (or other annotations). In other words, T(GDF) can be computed.

T(GDF) can be overlaid onto the determined plane or slice P as shown as a newly created composite image 2700 in FIG. 2. The newly created 2D composite image 2700 allows visualization of dose features known in relation to 2D image 2000 on P, a representative slice of 3D volume image data 2500 corresponding to the base image of 2D image 2000. T(GDF) may comprise the additional annotations or other information in addition to a transformation 2800 of the dosage information isodose lines 2200 and thus overlaying T(GDF) onto P may support labelling of the determined plane or slice P with a planned dose level and/or those additional annotations or other information.

Arrangements may allow a user, for example, a radiologist, to see geometric dose features that correspond to the representative CT planning image(s) overlaid onto newly acquired imaging information. For example, according to arrangements, a user can label a P image forming part of newly acquired imaging data, according to a previously planned and applied treatment dose. The labelling may allow the radiologist to distinguish between areas treated at various dose levels (as indicated by isodose lines). Overlaying isodose lines of a previous treatment onto a follow-up image can improve decision making of a radiologist.

Example Two: Previous Examination

Although an example has been described in relation to radiotherapy applied to a patient, representative 2D images from previous tumor assessment examination or imaging events can be treated similarly. FIG. 3 illustrates schematically input and output of a methodology according to an arrangement. According to such an approach, a 2D summary image of a previous examination or subject assessment is decomposed into a base image and an additional information image, where the additional information image comprises: detected and extracted annotations comprising at least one of: text or arrows or lines or colored region of interest (ROI) contour(s) or similar features of potential interest.

FIG. 3 shows a representative 2D image 3000 which can be extracted from storage medium. The 2D image may comprise a part of a patient report, a screen grab, part of an electronic medical record EMR or similar. In this instance, the 2D image may comprise an image annotation 3100. The image 3000 comprises a composite image formed from base image data and the additional information, in the form of annotation 3100. The base image includes one or more features 3200 of the region under study

According to a methodology in accordance with an arrangement, the representative 2D RGB image 3000 (of, for example, a previously acquired CT slice including additional information in the form of an image annotation) is decomposed into (i) a grey scale slice (S) representing the planning image and (ii) an additional information image (ADD). For example, colored annotations can be extracted from a representative 2D RGB image using a simple RGB decomposition of the 2D RGB image and detecting pixels with non-equal R, G, and B values.

FIG. 3 illustrates schematically a volume 3500 of 3D imaging data which is available in relation to a region under study. The volume of image data 3500 may comprise, for example a series of image "slices" obtained via appropriate CT or MRI techniques.

According to methods in accordance with arrangements, base image data from the 2D image 3000 and the 3D volume of the follow up image are processed so that a transformation T can be calculated. T maps S, the base image data in relation to 2D image 3000, into a 3D volume 3500 of a follow up image (V). T is calculated based upon a determination of a plane P in V which may be considered to correspond to representative slice S. The transformation T can be determined using slice to volume registration, or simple 2D to 2D registration, each technique operating with an aim of determining an index of a slice in V that best matches S. It will be appreciated that it may be impossible to find a slice of V 3500 which completely matches the features 3200 of the base image data of 2D image 3000. That may be because capture of the 2D image and 3D volume of image data is spaced apart in time and changes in features 3200 have occurred. In accordance with arrangements, a best fit slice or plane of the image data 3500 is found.

Once calculated, T can then be applied to the representative 2D additional information 3100 of 2D image 3000 (or other annotations). In other words, T(ADD) can be computed.

T(ADD) can be overlaid onto the determined plane or slice P as shown as a newly created composite image 3700 in FIG. 2. The newly created 2D composite image 3700 allows visualization of dose features known in relation to 2D image 3000 on P, a representative slice of 3D volume image data 3500 corresponding to the base image of 2D image 3000. T(ADD) may comprise the additional annotations or other information in addition to a transformation 3800 of the additional information and thus overlaying T(ADD) onto P may support labelling of the determined plane or slice P with those additional annotations or other information known in relation to 2D image 3000.

Arrangements may allow a user, for example, a radiologist, to see geometric dose features that correspond to the representative 2D images of a previous examination or assessment overlaid onto newly acquired imaging information. For example, according to arrangements, a user can label a P image forming part of newly acquired imaging data, according to a previously known annotation or measurement. The labelling may allow the radiologist to assess development of a disease or change to a region under study, thus supporting improved decision making of a medical professional.

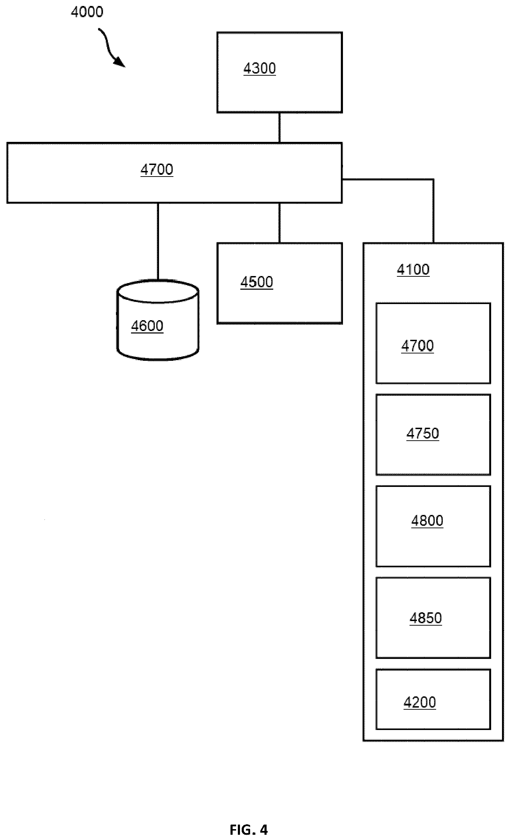

FIG. 4 is a schematic representation of a system for map additional information relating to a 2D image of a region of interest to a 3D image volume of the region of interest and producing a new composite 2D image of the region of interest as described or otherwise envisioned herein.

System 4000 includes one or more of: a processor 4100, memory 4200, user interface 4300, communications interface 4500, and storage 4600, interconnected via one or more system buses 4700.

In some embodiments, such as those where the system is part of, or in communication with, imaging hardware or an imaging platform, the hardware may include additional imaging hardware (not shown). It will be understood that FIG. 4 constitutes an abstraction of system 4000 and that the actual organization of the components of the system 4000 may differ from that shown.

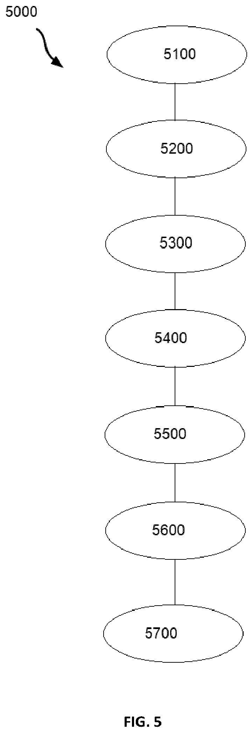

According to an embodiment, system 4000 comprises a processor 4100 capable of executing instructions stored in memory 4200 or storage 4600 or otherwise processing data. Processor 4100 may be configured to perform one or more steps of the method illustrated schematically in FIG. 5, and may comprise one modules described or otherwise envisioned herein. Processor 4100 may be formed of one or multiple modules, and can comprise, for example, a memory 4200. Processor 4100 may take any suitable form, including but not limited to: a microprocessor, microcontroller, multiple microcontrollers, circuitry, field programmable gate array (FPGA), application-specific integrated circuit (ASIC), a single processor, or a plurality of processors.

Memory 4200 can take any suitable form, including a non-volatile memory and/or RAM. The memory 4200 may include various memories such as, for example, a cache or system memory. As such, the memory 4200 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 4000. It will be apparent that, in embodiments where the processor implements one or more of the functions described herein in hardware, the software described as corresponding to such functionality in other embodiments may be omitted.

User interface 4300 may comprise one or more devices for enabling communication with a user such as an administrator, imaging or medical professional. The user interface may comprise any device or system that allows information to be conveyed and/or received, and may include a display, a mouse, and/or a keyboard for receiving user commands. In some embodiments, user interface 4300 may include a command line interface or graphical user interface that may be presented to a remote terminal via communication interface 4500. The user interface 4300 may be located with one or more other components of the system, or may located remote from the system and in communication via a wired and/or wireless communications network.

Communication interface 4500 may comprise one or more devices for enabling communication with other hardware devices. For example, communication interface 4500 may include a network interface card (NIC) configured to communicate according to the Ethernet protocol. Additionally, communication interface 4500 may implement a TCP/IP stack for communication according to the TCP/IP protocols. Various alternative or additional hardware or configurations for communication interface 4500 will be apparent.

Storage 4600 may include one or more machine-readable storage media such as read-only memory (ROM), random-access memory (RAM), magnetic disk storage media, optical storage media, flash-memory devices, or similar storage media. In various embodiments, storage 4600 may store instructions for execution by processor 4100 or data upon which processor 4100 may operate. For example, storage 4600 may store an operating system for controlling various operations of system 4000.

While system 4000 is shown as including one of each described component, the various components may be duplicated in various embodiments. For example, processor 4100 may include multiple microprocessors that are configured to independently execute the methods described herein or are configured to perform steps or subroutines of the methods described herein such that the multiple processors cooperate to achieve the functionality described herein. Further, where system 4000 is implemented in a cloud computing system, the various hardware components may belong to separate physical systems. For example, processor 4100 may include a first processor in a first server and a second processor in a second server. Many other variations and configurations are possible.

According to an embodiment, processor 4100 comprises one or more modules to carry out one or more functions or steps of the methods described or otherwise envisioned herein. For example, processor 4100 may comprise: a decomposition module 4700 a slice assessment module 4750, a transformation module 4800, and/or an image production module 4850.

FIG. 5, is a flowchart of a method 5000 for mapping additional information relating to a 2D image of a region of interest to a 3D image volume of the region of interest and producing a new composite 2D image of the region of interest.

At step 5100, a system for such mapping and image generation provided. The system may be any of the systems described or otherwise envisioned herein, and may comprise any of the components or modules described or otherwise envisioned herein.

At step 5200 of the method, one or more 2D composite image of the region of interest is received by the system or provided to the system.

At step 5300 of the method, a 3D image volume of the region of interest is received by the system or provided to the system.

At step 5400 of the method, the system is configured, for example, via steps taken by a decomposition module, to decompose the 2D composite image into at least: a base image; and an additional image comprising additional information.

At step 5500 of the method, the system is configured, for example via steps taken by a slice assessment module, to identify, based on an assessment of the base image and the 3D image volume, a representative 2D slice of the 3D image volume which corresponds to the base image.

At step 5600 of the method, the system is configured, for example via steps taken by a transformation module, to evaluate a transformation of the base image which maps the base image to the representative 2D slice.

At step 5700 of the method, the system is configured to, for example, via steps taken by an image production module, to combine the representative 2D slice with an image formed by applying the evaluated transformation to the additional image to produce a new composite 2D image of the region of interest.

A person of skill in the art would readily recognize that steps of various above-described methods can be performed by programmed computers. Herein, some embodiments are also intended to cover program storage devices, e.g., digital data storage media, which are machine or computer readable and encode machine-executable or computer-executable programs of instructions, wherein said instructions perform some or all of the steps of said above-described methods. The program storage devices may be, e.g., digital memories, magnetic storage media such as a magnetic disks and magnetic tapes, hard drives, or optically readable digital data storage media. The embodiments are also intended to cover computers programmed to perform said steps of the above-described methods. The tern non-transitory as used herein, is a limitation of the medium itself (i.e., tangible, not a signal) as opposed to a limitation on data storage persistency (e.g. RAM vs ROM).

Although example embodiments of the present invention have been described in the preceding paragraphs with reference to various examples, it should be appreciated that modifications to the examples given can be made without departing from the scope of the invention as claimed.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

Whilst endeavoring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid state medium supplied together with, or as part of, other hardware, but may also be distributed in other forms, such as via the Internet or other wired or wireless telecommunication systems. Any reference signs in the claims should not be construed as limiting the scope.

**Claims**

1.  An apparatus (4000) configured to map additional information relating to a 2D image of a region of interest to a 3D image volume of the region of interest, the apparatus comprising:

    at least one processor (4100); and
    at least one memory (4600, 4200) storing instructions that, when executed by the at least one processor, cause the apparatus at least to:

    receive (5200) a 2D composite image of the region of interest;
    receive (5300) a 3D image volume of the region of interest;
    decompose (5400) the 2D composite image into at least:

    a base image; and
    an additional image comprising the additional information;

    identify (5500), based on an assessment of the base image and the 3D image volume, a representative 2D slice of the 3D image volume which corresponds to the base image;
    evaluate (5600) a transformation of the base image which maps the base image to the representative 2D slice; and
    combine (5700) the representative 2D slice with an image formed by applying the evaluated transformation to the additional image to produce a new composite 2D image of the region of interest.

2.  Apparatus according to claim 1, wherein the 2D image of a region of interest comprises: a color image.

3.  Apparatus according to claim 1 or claim 2, wherein the composite 2D image comprises: a 2D summary image of a 3D image volume representative of the region of interest.

4.  Apparatus according to any preceding claim, wherein the composite 2D image comprises: additional information relating to a treatment or procedure performed upon the region of interest.

5.  Apparatus according to any preceding claim, wherein the additional information comprises: a geometrical distribution of a therapy dosage applied to the region of interest.

6.  Apparatus according to claim 5, wherein the therapy comprises radiotherapy.

7.  Apparatus according to claim 5 or claim 6, wherein the geometrical distribution comprises: isodose lines or color mapped dosage information.

8.  Apparatus according to any preceding claim, wherein the additional information comprises: one or more annotation comprising: an outline of a feature of interest; or a marker of a feature of interest; or a measurement of a feature of interest.

9.  Apparatus according to any preceding claim, wherein decomposing the 2D composite image (2000; 3000) comprises: decomposing the 2D composite image into at least: a greyscale base image; and an additional image comprising the additional information (2100; 3100).

10. Apparatus according to any preceding claim, wherein decomposing the 2D composite image comprises: analysis of pixels of the 2D composite image to determine the greyscale base image and an additional image comprising the additional information.

11. Apparatus according to any preceding claim, wherein identifying a representative 2D slice (2700; 3700) of the 3D image volume (2500; 3500) which corresponds to the base image comprises assessing one or more plane image forming the 3D image volume against the base image and selecting a plane image of the assessed one or more plane image that best matches the base image.

12. Apparatus according to any preceding claim, wherein identifying the representative 2D slice (2700; 3700) of the 3D image volume (2500; 3500) and evaluating a transformation of the base image which maps the base image to the representative 2D slice comprises using a slice to volume or 2D to 2D registration image registration technique.

13. Apparatus according to any preceding claim, wherein evaluating a transformation of the base image which maps the base image to the representative 2D slice (2700; 3700) comprises evaluating one or more difference between those images resulting from one or more of: rotation, translation, deformation, or scaling.

14. A computer implemented method (5000), for mapping additional information relating to a 2D image of a region of interest to a 3D image volume of the region of interest, the method comprising:

   receiving (5200, 5300) a 2D composite image of the region of interest and a 3D image volume of the region of interest;
   decomposing (5400) the 2D composite image into at least:

      a base image; and
      an additional image comprising the additional information;

   identifying (5500), based on an assessment of the base image and the 3D image volume, a representative 2D slice of the 3D image volume which corresponds to the base image;
   evaluating (5600) a transformation of the base image which maps the base image to the representative 2D slice; and
   combining the representative 2D slice with an image formed by applying the evaluated transformation to the additional image to produce (5700) a new composite 2D image of the region of interest.

15. A computer program product operable, when executed on a computer, to perform the method of claim 14.

**FIG. 1A**

**FIG. 1B**

FIG. 2

**FIG. 3**

4000

4300

4700

4600

4500

4100

4700

4750

4800

4850

4200

FIG. 4

5000

```
        5100

        5200

        5300

        5400

        5500

        5600

        5700
```

FIG. 5

Europäisches
Patentamt

European
Patent Office

Office européen
des brevets

# EUROPEAN SEARCH REPORT

Application Number

EP 22 21 0590

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 3 264 298 A1 (EBIT SRL [IT]) 3 January 2018 (2018-01-03) * abstract * * figures 7B,10A-10D * * paragraphs [0005] - [0007], [0080], [0099] - [0101] * | 1-15 | INV. G06T11/60 |
| A | SAVJANI RICKY R. ET AL: "A Framework for Sharing Radiation Dose Distribution Maps in the Electronic Medical Record for Improving Multidisciplinary Patient Management", RADIOLOGY: IMAGING CANCER, vol. 3, no. 2, 1 March 2021 (2021-03-01), page e200075, XP093009270, ISSN: 2638-616X, DOI: 10.1148/rycan.2021200075 * abstract * * figures 1,2 * * page 3, left-hand column * * p. 2-3 bridging par. * | 1-15 | |
| A | WO 2020/210679 A1 (UNIV LELAND STANFORD JUNIOR [US]) 15 October 2020 (2020-10-15) * abstract * * page 1 - page 2 * * page 5, paragraph 1 * * page 19, paragraph 4 * | 1-15 | TECHNICAL FIELDS SEARCHED (IPC) G06T G06K G06V |

The present search report has been drawn up for all claims

| Place of search | Date of completion of the search | Examiner |
|---|---|---|
| Munich | 4 May 2023 | Scholz, Volker |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 21 0590

This annex lists the patent family members relating to the patent documents cited in the above-mentioned European search report.
The members are as contained in the European Patent Office EDP file on
The European Patent Office is in no way liable for these particulars which are merely given for the purpose of information.

04-05-2023

| Patent document cited in search report | | Publication date | Patent family member(s) | | Publication date |
|---|---|---|---|---|---|
| EP 3264298 | A1 | 03-01-2018 | NONE | | |
| WO 2020210679 | A1 | 15-10-2020 | EP | 3953863 A1 | 16-02-2022 |
| | | | US | 2022198689 A1 | 23-06-2022 |
| | | | WO | 2020210679 A1 | 15-10-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SAVJANI et al.** A Framework for Sharing Radiation Dose Distribution Maps in the Electronic Medical Record for Improving Multidisciplinary Patient Management. *Radiol Imaging Cancer,* 12 March 2021, vol. 3 (2), e200075